(19) Europäisches Patentamt
European Patent Office
Office européen des brevets

(11) **EP 4 360 545 A1**

(12) **EUROPEAN PATENT APPLICATION**

(43) Date of publication:
**01.05.2024 Bulletin 2024/18**

(21) Application number: **22204008.1**

(22) Date of filing: **27.10.2022**

(51) International Patent Classification (IPC):
*A61B 5/024* (2006.01)    *A61B 5/11* (2006.01)
*G16H 20/30* (2018.01)    *A61C 17/22* (2006.01)
*B26B 19/38* (2006.01)    *A61B 5/0533* (2021.01)
*A61B 5/08* (2006.01)    *A61N 5/06* (2006.01)
*A61B 5/16* (2006.01)    *A61B 5/06* (2006.01)

(52) Cooperative Patent Classification (CPC):
**A61B 5/4824; A61B 5/0053; A61B 5/02438;
A61B 5/0533; A61B 5/1104; A61B 5/445;
A61B 5/4547; A61B 5/682; A61B 5/6843;
A61B 5/6898; A61B 5/7246; A61B 5/7278;
A61B 5/7282; G16H 20/30; G16H 40/63;** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **RMAILE, Amir Hussein
Eindhoven (NL)**

• **JOHNSON, Mark Thomas
Eindhoven (NL)**
• **GERHARDT, Lutz Christian
Eindhoven (NL)**
• **SPELT, Hanne Adriana Alijda
Eindhoven (NL)**
• **KOOIJMAN, Gerben
Eindhoven (NL)**
• **PERRONE, Antonio Luigi
Eindhoven (NL)**

(74) Representative: **Philips Intellectual Property &
Standards
High Tech Campus 52
5656 AG Eindhoven (NL)**

(54) **PAIN METRIC**

(57)    A means of deriving an individualized calibration or relationship between two different measurable correlates of pain, wherein one relates to a behavioral response of the individual to a sensation of pain, measured using sensing data obtained from a personal care device, and the other relates to a different pain response output, either a physiological response or a perceptual response.

FIG. 3

**(Cont. next page)**

(52) Cooperative Patent Classification (CPC): (Cont.)
   **G16H 50/20;** A61B 5/0022; A61B 5/0057;
   A61B 5/02405; A61B 5/02416; A61B 5/065;
   A61B 5/0816; A61B 5/1128; A61B 5/162;
   A61B 5/4821; A61B 5/4839; A61B 2560/0223;
   A61B 2562/0219; A61B 2562/0247;
   A61H 2230/06; A61H 2230/65; A61N 5/0616;
   B26B 19/388

**Description**

FIELD OF THE INVENTION

The present invention relates to a system and method for measuring user pain.

BACKGROUND OF THE INVENTION

Pain is a complex process with sensory and emotional components.

Pain can be categorized into acute pain and chronic pain. Acute pain is a symptom of acute or potential tissue damage.

With regards to acute pain, this has an afferent part (which relates to a pain signal from the physical stimulus at the tissue site, through the peripheral nervous system and the spine to the brain) and an efferent part (which relates to the pathway from the brain to pain perception).

With regards to the afferent part, this may be understood more fully as follows. Acute pain is a sensation that can occur when a physical stimulus affects a specific type of sensory receptor (i.e. nociceptor or pain receptor) at a tissue site. Pain often involves direct contact with e.g., skin, internal organs, or the musculoskeletal system. The nociceptors detect the painful stimulus and transmit a pain signal to the spinal cord. The spinal cord sends the signal to the brain. With regards to the efferent part, in the brain, various areas become activated including the somatosensory cortex (responsible for processing physical sensations).

It is possible to measure different outcomes of this process.

In particular, various automatic involuntary responses occur within the biology of the person (both body and mind) in response to a pain event.

One class of reaction is physiological reaction, wherein various physiological systems become activated responsive to the pain event. For example, among other things, it leads to activation of the electrodermal system. This can be sensed through measurement of galvanic skin responses (GSR). It also leads to a variety of other physiological responses such as changes in heart rate and heart rate variability (HRV). The level of physiological activation may differ between subjects due to differing physiological reactivity. Physiological reactivity relates to physiological changes an individual demonstrates in response to a stimulus or in a given situation. For some people these changes are more extreme than for others. In addition, reactivity to a similar stimulus may differ within a person depending upon baseline activity (e.g. after physical effort the baseline is higher, resulting in lower reactivity). Reference is made to the following handbook which explains physiological reactivity with greater detail: Cacioppo, J. T., Tassinary, L. G., & Berntson, G. G. (2007). The handbook of psychophysiology. In J. T. Cacioppo, L. G. Tassinary, & G. G. Berntson (Eds.), Cambridge University Press (3rd ed., Vol. 44). Cambridge University Press.

Another class of response is the eliciting of a perceptual experience of pain, i.e. the subject perceives the painful stimulus responsive to activation of particular brain areas. This can be probed through pain-response surveys which ask an individual to rate their perceived level of pain. Pain perception involves several psychological processes, including attentional orienting to the painful sensation and its source, cognitive appraisal of the meaning of the sensation, and the subsequent emotional, psychophysiological, and behavioral reaction. All of these processes feed back to influence pain perception. As people may differ in these psychological processes, the amount of suffering due to a particular stimulus varies between subjects. Suffering is an unpleasant experience associated with negative cognitive, emotional, and autonomic responses to a stimulus.

The difference between individuals in the physiological reactivity to pain and in pain perception may be referred to collectively as pain reactivity. The two are linked in that they are both essentially involuntary responses to pain elicited in the person: one being physiological reaction, and one being a conscious experiential response.

In addition to involuntary/automated responses in the body and mind of the person, pain events also give rise to behavioral or action responses by the person. For example, responsive to a certain stimulus inducing a pain signal at a tissue site, a person will typically perform a bodily action in response, e.g. to attempt to remove the stimulus. The proneness of a person to react behaviorally to a certain pain stimulus is referred to as pain sensitivity.

In the context of the present disclosure, pain sensitivity is intended to refer to the proneness of a person to react to a given physical stimulus which elicits a given neural pain signal at the tissue site. In other words, pain sensitivity describes the degree to which a user reacts to a standardized pain stimulus. In an idealized model, the pain sensitivity measure would be a measure of a degree of user reaction or response to a standardized neural pain signal elicited at a tissue site responsive to an applied physical stimulus. Put in other terms, it could be characterized as the level of pain stimulus required before a person reacts. The physical stimulus could be an external, experimentally applied stimulus or a stimulus arising from a pathology at the tissue site.

It is expected that there will be individual differences between persons in pain sensitivity. There may furthermore occur in an individual changes over time in pain sensitivity, due to sensitization. Upon repeated exposure to a certain stimulus, a person can become less prone to react to it in future. Sensitization can occur at the level of the nociceptor (peripheral sensitization) or the level of the spinal cord or brain (central sensitization). Additionally, individuals may differ as to when nerves fire responsive to a particular physical stimulus.

Thus, it can be understood from the above that a particular pain response output is characterized by at least two contributing factors: the person's pain reactivity (degree of neural or neurological reaction to a pain stimulus in the body and mind), and the person's pain sensitivity (proneness to react to a given pain stimulus).

Measuring acute pain responses in a person is of value since pain is typically indicative of injury or pathology. Given the complexity of the pain response mechanisms in the body, it is a challenge to obtain pain information for an individual which can be relied upon to give an objective, comparable indicator which can be used in standardized monitoring and treatment workflows.

It would be of benefit to provide improvements in the area of pain detection and monitoring that allow for more reliable and objective measurement and monitoring of individual pain. Reference is made to the following papers for further background information:

De Ridder, D., Adhia, D., & Vanneste, S. (2021). The anatomy of pain and suffering in the brain and its clinical implications. Neuroscience & Biobehavioral Reviews, 130, 125-146.
Garland, E. L. (2012). Pain processing in the human nervous system: a selective review of nociceptive and biobehavioral pathways. Primary Care: Clinics in Office Practice, 39(3), 561-571.

SUMMARY OF THE INVENTION

Motivating the present invention is a recognition by the inventors that one way to target a more objective probing of an individual's pain activity is to measure two parameters known to represent measurable response outputs to physical pain-inducing stimuli, but wherein the two parameters differ in the particular neural route through which the response is generated. In particular, it is the recognition of the inventors that a particularly powerful pair of measurable parameters would be: a first parameter whose magnitude is known to be affected/modulated more by a person's pain sensitivity than the persons pain reactivity, and a second parameter whose magnitude is affected/modulated more by a person's pain reactivity that then person's pain sensitivity. In this way, the two measured parameters provide complementary information which, in combination, provides a more objective indicator of a person's pain activity than a single measurable parameter alone.

The invention is defined by the claims.

According to examples in accordance with an aspect of the invention, there is provided a method, comprising: obtaining sensor data from one or more sensors integrated in a personal care device indicative of personal care action by the user using the device over the course of one or more personal care sessions; determining a first metric of user pain experienced during the one or more personal care session based on analysis of patterns in the sensor data; receiving further data, the further data comprising biological parameter sensor data acquired over the course of said one or more personal care sessions, or user pain-report data corresponding to said one or more personal care sessions; determining a second metric of user pain experienced during the one or more personal care sessions based on the further data; deriving a pain calibration metric based on a relationship between the first and second metrics of estimated user pain. Preferably, the method further comprises generating a data item indicative of the pain calibration metric.

With regards to the first pain metric, here the idea is to efficiently utilize sensors (e.g. force and/or motion sensors) integrated in a personal care device (such as a powered toothbrush or shaver) that measure parameters under the control of a user in order to indirectly infer user pain levels. As will be explained below, the inventors have realized that patterns in such sensor data can be used to infer user personal care behavior patterns over each personal care session and that these behavior patterns can be used to infer user experiences of pain. There are multiple different ways of doing this, as will become clear. For instance, it will be apparent that when a user feels pain, an instinctive motion response often follows, and thus it will be readily appreciated how the data of motion or force sensors might be used to detect this. The motion response could be reflexive or it could arise from a consciously controlled action. Likewise, it will be recognized that applied pressure and patterns of scrubbing are typically different in anatomy areas which are painful compared to those which are not. Thus, the inventors have recognized that user pain can very efficiently and effectively be monitored through patterns in personal care device sensor data. With regards to the personal care action, this may generally refer to action by the user using the personal care device involving physical engagement/interaction of the personal care device with the user's body.

With regards to the second pain metric, here it is proposed to: measure physiological parameters which are known to correlate with pain, such as skin conductance, heart rate or heart rate variability, respiration rate, facial expression, among others; and/or to record a user's conscious perceptual experience of pain through the form of pain reports.

It is the recognition of the inventors that the first pain metric is more heavily weighted, in terms of its strength, by a person's pain sensitivity than by a person's pain reactivity. As explained above, pain sensitivity is the proneness of a person to react behaviorally to a certain pain stimulus. Thus, a pain metric derived from sensor data indicative of user operation (e.g. movement or application) of the personal care device will be influenced to a relative greater degree by the user's pain sensitivity (pain threshold).

It is the further recognition of the inventors that the second pain metric is more heavily weighted, in terms of its strength, by a person's pain reactivity than a person's pain sensitivity. As discussed above, pain reactivity relates to a strength of response (neural or perceptual) that arises involuntarily in the user, and this is more directly probed by pain metrics such as physiological parameter measurements and user pain experience.

Thus, it is the further recognition of the inventors that a further metric computed as a function of the two, and preferably which defines a relationship or mapping between the two, can provide a valuable indicator of a person's individualized pain response characteristics. At least one ultimate technical benefit of this arises from the fact that pain, as a neural response mechanism, is a very close proxy of tissue health or injury. Thus, providing a means of obtaining a more objective measure of a user's pain activity during the personal care session provides a more objective insight or measure of the underlying health or injury of the relevant tissue whose physical stimulation triggered the pain response(s).

The first and second pain metrics may each either comprise a single-value (scalar) metric or may each be a function of time (i.e. a time-dependent pain metric). In the case that it is a single value metric, it can be derived in some examples by first obtaining a pain signal which is a function of time (e.g. GSR or inverse of application pressure) and then extracting or synthesizing from that a single value metric (e.g. an average or maximum value). With regards to the first pain metric in particular, in some examples, obtaining a pain signal which is a function of time may be done simply through measuring a time series of values from a sensor over a time period (e.g. taking an inverse of measured device application pressure), or it may be constructed. In the latter case, for example, deriving the first pain metric may comprise: detecting occurrence one or more discrete pain events over a personal care session based on detecting one or more pre-defined waveform features (e.g. spikes or drops) in a sensor waveform; and constructing a pain signal by plotting the pain events as a function of time to form a pain event time series. Each pain event may have an associated time and magnitude. This constructed pain signal could be used as the first pain metric, yielding a time-dependent first pain metric. Alternatively, from this might be extracted a single-value first pain metric (e.g. from an average or maximum value of the plotted pain event points). These options will be explained in more detail to follow.

In some embodiments, the determining the pain calibration metric comprises determining a transfer function which defines a mapping between the first and second metrics of user pain.

In some embodiments, the determining the pain calibration metric comprises determining a ratio between the first and second metrics of user pain. This provides one simple estimation of the relationship between the two pain metrics.

As mentioned above, in some embodiments, the first and/or second metric is derived as a single value or vector which may for example be extracted or synthesized from a time-dependent pain signal.

Thus, in some embodiments, the determining the first metric of user pain comprises processing the sensor data to determine a signal of user pain as a function of time over each personal care session ('first pain signal'), and extracting a (e.g. single-value) first pain metric based on processing of said first pain signal. In some embodiments, the determining the second metric of user pain comprises processing the sensor data to determine a signal of user pain as a function of time over each personal care session ('second pain signal'), and extracting a (e.g. single-value) second pain metric based on processing of said second pain signal. In some embodiments, each of the first and second pain signals might be normalized before extracting the first and second pain metrics. In some embodiments, each of the first and second pain signals might be first processed to remove the DC bias component of the signal, leaving just the AC component. For example, the signals might each be high-pass filtered.

In some embodiments, the determining the first metric of user pain comprises processing the sensor data to determine a signal of user pain as a function of time over each personal care session, and determining a maximum value of said signal of user pain for each personal care session, and using said maximum value or an average thereof over the one or more personal care sessions as the first metric of user pain.

In some embodiments, the determining the second metric of estimated user pain comprises processing the biological parameter sensor data to determine a further signal of user pain as a function of time over each personal care session, and determining a maximum value of said further signal of user pain for each personal care session, and using said maximum value or an average thereof over the one or more care sessions as the second metric of user pain.

In other words, the calibration metric is derived based on comparing maximum values of the two pain measures.

In some embodiments, the determining of the first metric of user pain comprises processing the sensor data to determine a signal of user pain as a function of time over each personal care session, and determining an average value of said signal of user pain for each personal care session, and using said average value as the first metric of user pain.

In some embodiments, the determining the second metric of estimated user pain comprises processing the biological parameter sensor data to determine a further signal of user pain as a function of time over each personal care session, and determining an average value of said further signal of user pain for each personal care session, and using said average value over the one or more care sessions as the second metric of user pain.

In other words, in this set of embodiments, it is proposed to compute an average of each pain signal, and wherein the calibration metric is derived based on a relationship between these averages.

In some embodiments, as mentioned above, each of the pain metrics is based on an underlying pain signal which has a value as a function of time. The two underlying pain signals may have a time offset between them which may have an underlying physiological cause, e.g. a difference in the pathway between the one measurable correlate of pain and the other measurable correlate of pain. In some embodiments, the method may comprise determining a time offset between the two pain signals.

In some embodiments, the method comprises computing a cross-correlation between the pain signals underlying the two pain metrics. In some embodiments, this may be used to determine the time offset between the two pain signals.

For example, in some embodiments, the determining the first metric of user pain comprises processing the sensor data to determine a signal of user pain as a function of time over each personal care session. The determining the second metric of user pain may comprise processing the sensor data to determine a further signal of user pain as a function of time over each personal care session. The determining the calibration metric may comprise, for at least one of the one or more personal care sessions, computing a cross-correlation between the first and second signals of user pain and determining from the cross-correlation a time offset between the first and second pain signals.

In some embodiments, the method may comprise adjusting a time-registration between the first and second pain signals to compensate for the determined time offset. In other words, it is proposed to add a time offset to one or both signals in such a way as to reduce or substantially eliminate the time asynchrony between the two. In some embodiments, the method may then further comprise one of the processes described above wherein a single-value pain metric is extracted from each of the (now time-synchronized) pain signals.

In some embodiments, the method further comprises receiving position sensor data, and wherein the method comprises determining a respective first and second metric of estimated user pain for different respective bodily locations.

In some embodiments, the method further comprises computing an average of the first pain metrics for the different locations and an average of the second pain metrics for the different locations, and computing the calibration metric based on a relationship between said average first pain metric and average second pain metric.

In some embodiments, the method further comprises computing a respective calibration metric for said different respective bodily locations.

In some embodiments, the biological parameter sensor is a skin conductance sensor, such as a galvanic skin response (GSR) sensor.

In some embodiments, the one or more sensors integrated in the personal care device may include: one or more force and/or motion sensors; and/or one or more operational signal sensors adapted to output an operational signal indicative of an operational parameter of the personal care device which correlates with physical interaction of the personal care device with bodily surfaces.

In some embodiments, the personal care device is an oral care device.

In some embodiments, the first metric of user pain is determined based on detecting one or more inflections in at least one sensor signal waveform included in the sensor data, the one or more inflections being indicative of user pain response events during the given personal care session.

In some embodiments, the sensor data includes a pressure sensor waveform indicative of application pressure of a portion of the personal care device to surfaces during use, and wherein the first metric of user pain is determined based on the pressure signal.

In some embodiments, the computing the first pain metric comprises computing a time-dependent first pain signal, and wherein the first pain signal is inversely proportional to said application pressure of a portion of the personal care device to surfaces during use. For example, the first pain signal, pain1(t), might be defined as pain1(t) = c/P(t), where P(t) is said measure of application pressure of the personal care device to the body as a function of time, and c is a constant (which could in a simple case simple be set equal to 1). This is based on the recognition that pain is inversely correlated with application pressure P(t) because a user will tend to press less hard when they feel pain and vice versa.

In some embodiments, the first metric of user pain may be determined based on detecting one or more characteristic pattern features in a sensor signal waveform, each detected pattern feature corresponding to a detected pain event. In some embodiments, the computing the first pain metric comprises constructing a first pain signal as a function of time by plotting the detected pain events as a function of time. This plot may be used as the first pain metric, yielding a time-dependent first pain metric. Alternatively, a single-value pain metric might be computed or extracted from the plot, e.g. an average or maximum value.

In some embodiments, the first metric of user pain may be determined based on detecting valleys in the pressure sensor waveform, indicative of a drop in applied pressure during a sub-interval of a given personal care session. Each detected valley may correspond to a pain event.

In some embodiments, the sensor data includes a motion sensor signal, and wherein the first metric of user pain is determined based on detecting inflections in motion, indicative of user reaction to locally experienced pain. Each detected inflection may correspond to a pain event.

In some embodiments, the sensor data includes a motion sensor signal and wherein the first metric of user pain is determined based on detecting in the motion sensor signal periods of alternating motion, indicative of a scrubbing action by the user, and wherein an amplitude or time duration of the periods of alternating motion are used to infer a level of patient comfort. Each signal period of alternating motion may correspond to a pain event.

The calibration metric allows for calibration to be performed of a pain metric obtained in further personal care sessions.

For example, another aspect of the invention provides a method for calibrating pain measurement information for a user. The steps of this method may be performed as part of a method as already outlined above, or may be performed as a separate method.

This method may comprise receiving input data, the input data comprising biological parameter sensor data acquired during use of a personal care device over the course of one or more personal care sessions, or user pain-report data corresponding to said one or more personal care sessions, and determining a measure of estimated user pain experienced during the one or more personal care session based on the input data. Alternatively, the method may comprise receiving input data, the input data comprising sensor data from one or more sensors integrated in a personal care device indicative of personal care action by the user using the device over the course of one or more personal care sessions, and determining a measure of estimated user pain experienced during the one or more personal care session based on analysis of patterns in the sensor data;

The method may further comprise retrieving from a datastore a pre-derived pain calibration metric for the user.

The method may further comprise applying the pain calibration metric to the measure of estimated user pain to derive a calibrated measure of estimated user pain.

The method may further comprise generating a data item indicative of the calibrated measure of estimated user pain.

A further aspect of the invention is a processing device adapted to perform a method in accordance with any example or embodiment described in the disclosure or in accordance with any claim of this application.

For example, according to at least one or more embodiments, there is provided a processing device comprising: an input/output; and one or more processors. The one or more processors may be configured to: obtain sensor data from one or more sensors integrated in a personal care device indicative of personal care action by the user using the device over the course of one or more personal care sessions; determine a first metric of user pain experienced during the one or more personal care session based on analysis of patterns in the sensor data; receive further data, the further data comprising biological parameter sensor data acquired over the course of said one or more personal care sessions, or user pain-report data corresponding to said one or more personal care sessions; determine a second metric of user pain experienced during the one or more personal care sessions based on the further data; and derive a pain calibration metric based on a relationship between the first and second metrics of estimated user pain. Optionally the one or more processors are further configured to generate a data item indicative of the pain calibration metric and optionally routing the data item to the input/output.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an expected differing application pressure behavior of a personal care device for a person with high pain sensitivity as compared with a person with low pain sensitivity
Fig. 2 illustrates an expected differing physiological response of a person with high pain reactivity as compared with a person with low pain reactivity;
Fig. 3 illustrates an example method in accordance with one or more embodiments of the invention;
Fig. 4 illustrates components of an example processing device and system according to one or more embodiments.
Fig. 5 illustrates neural pathways of pain signals though the nervous system;
Fig. 6 illustrates an expected progression of first and second pain metrics following a pain-causing physical stimulus;
Fig. 7 illustrates recorded brushing action sensor data in the form of a brushing pressure signal which might be used to infer user pain from a valley in the brushing pressure signal;
Fig. 8 illustrates recorded brushing action sensor data in the form of motion data in a direction away from a surface of a tooth which might be used to infer user pain from an upward inflection, or spike, in the signal;
Fig. 9 illustrates recorded brushing action sensor data in the form of motion data in a direction in-plane with a surface of a tooth, and illustrates how user pain might be inferred from a reduced amplitude in an alternating scrubbing motion pattern; and

Fig. 10 and Fig. 11 illustrate comparative example plots of average brushing pressure over a monitoring period which includes a period prior to a treatment and following a treatment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means of deriving an individualized calibration or relationship between two different measurable correlates of pain, wherein one relates to a behavioral response of the individual to a sensation of pain, measured using sensing data obtained from a personal care device, and the other relates to a different pain response output, either a physiological response or a perceptual response.

These two different response outputs are generated through different pathways of the nervous system, and thus provide different windows onto the individual's personal pain response characteristics. By deriving this person-specific relationship between the two, this yields a highly personalized metric, referred to in this document as a calibration metric, which is informative about the patient's pain response characteristics. This metric in a most simple case could be used to map one measured pain metric to another. It could additionally or alternatively be used to modulate or adjust or normalize a single measured pain metric. It could additionally or alternatively be used as an informative biological parameter in its own right which could, for instance, inform decisions about treatment or monitoring of the person.

There are multiple different means for measuring pain.

One method employs self-report measures such as visual analogue scales (VAS) to assess pain. Another method to assess pain is through measurement of physiological pain responses. In particular, various biological signals are known to correlate with pain, and can be used as indicators of pain or stress. For example, electrodermal activity, i.e., sweating, has been linked to pain experiences. Heart rate variability is also known to be indicative of pain experiences.

Pain sensitivity is the proneness of a person to react to a pain-inducing physical stimulus, and it varies widely between subjects. A lower pain sensitivity means that the person can withstand more of a certain trigger that causes pain before a person will rate the pain as high or act on it compared to someone else. Most indicative for pain sensitivity are parameters that relate to the pain but are under control of the user (for example brushing parameters), as the user will act based on the experienced pain.

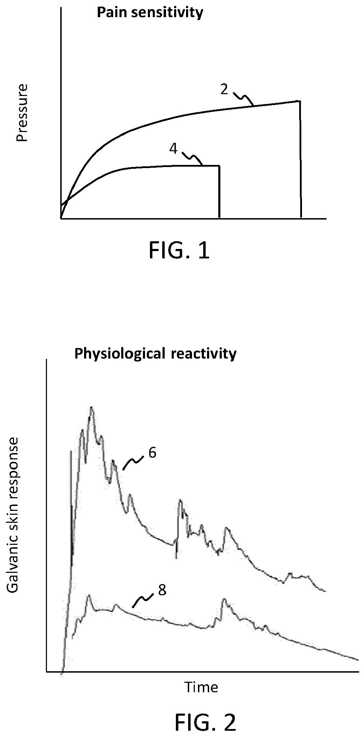

Fig. 1 schematically illustrates this concept. Fig. 1 illustrates a simulated pressure curve, indicating application pressure of a personal care device to a bodily surface (e.g. an oral care device to a tooth surface) which is injured and is a source of pain. It will be understood that application pressure of the personal care device is inversely related to experienced pain since a person will typically reduce pressure when they experience pain. Fig. 1 shows a first curve (labelled line 2) which represents an application pressure curve for an individual with lower pain sensitivity, and a second curve (labelled line 4) which represents an application pressure curve for an individual with higher pain sensitivity. For this illustration it is assumed that the physical stimulus and the level of tissue injury is identical for both persons. It can be seen that the individual with higher pain sensitivity can handle less pain for a shorter time before reacting.

Pain reactivity expresses the magnitude of a subjective pain response to a pain-triggering physical stimulus. As explained above, it is mediated at the level of the brain, after the pain signal has been generated and travelled through the peripheral nervous system and the spine. The subjective pain response includes both involuntary physiological responses (e.g. in the skin conductance system or heart rate system) and responses in the perceptual system (i.e. conscious experience of pain). Regarding the physiological reactivity, this involves any bodily changes in response to a trigger/stimulus, and this also varies between subjects. For example, some people have a general tendency to sweat more than others, e.g. because their sweat glands are more active or simply because they have a higher density of sweat glands. Several measurable biosignals, including skin conductance and heart rate variability (HRV) have been found to be indicative of pain.

Fig. 2 illustrates the concept of pain reactivity. Fig. 2 illustrates a simulated galvanic skin response (GSR) curve, indicating a GSR response of a person upon physical stimulation of a tissue site which is injured and is a source of pain. Fig. 2 shows a first curve (labelled line 6) which represents a GSR curve for an individual with higher pain reactivity, and a second curve (labelled line 8) which represents a GSR curve for an individual with lower pain reactivity. For this illustration, it is assumed that the physical stimulus and the level of tissue injury is identical for both persons. It can be seen that the individual with lower pain reactivity has a smaller magnitude of response in the skin conductance system than the individual with higher pain reactivity.

It is the general proposition according to embodiments of the present invention to capture two different parameters, each of which provides a proxy indicator of pain, but wherein each parameter is generated via a different pathway, and wherein one is more heavily influenced (in terms of its magnitude) by pain sensitivity, while the other is more heavily influenced (in terms of its magnitude) by pain reactivity.

By way of illustration, at least one advantageous set of embodiments may include one or more of the following features:

- A means to capture personal care device parameters for use as pain proxy values over a certain time interval (e.g., pressure, motion, position). These feed into the first pain metric.
- A means to record a second measure of pain, e.g. self-reported pain (e.g. in the form of the visual analog scale), or a bio-signal measured over a certain time interval, e.g. galvanic skin response or heart rate variability.
- A processor running an algorithm that: receives input from both aforementioned means, and uses a relationship of both means to determine a calibration factor between the two.

In some embodiments, the derived patient-specific calibration factor could be used subsequently in any personal care device, for instance for use in guiding or configuring elements of patient health care, e.g. oral health care, e.g., redirect a patient to see a specialist, general physician, dental practitioner.

Embodiments of the present invention may provide several advantageous technical contributions. At least a first is the provision, in the form of a pain calibration metric, of a personalized metric indicative of pain response characteristics of a person. A second is the ability to calibrate pain measurements obtained in future for the same person, by applying the calibration metric. This yields more objective, replicable and therefore reliable pain measurements. This allows, for example, for remote monitoring of pain using a personal care device in a way that is reliable and objective.

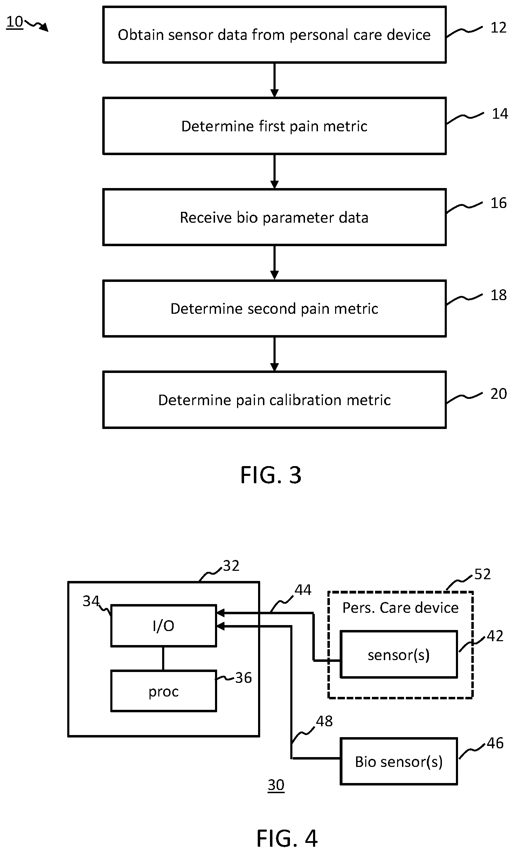

Fig. 3 outlines in block diagram form steps of an example computer implemented method 10 according to one or more embodiments. The steps will be recited in summary, before being explained further in the form of example embodiments.

The method 10 comprises obtaining 12 sensor data from one or more sensors integrated in a personal care device indicative of personal care action by the user using the device over the course of one or more personal care sessions.

The method 10 further comprises determining 14 a first metric of user pain experienced during the one or more personal care session based on analysis of patterns in the sensor data.

The method 10 further comprises receiving 16 further data, the further data comprising biological parameter sensor data acquired over the course of said one or more personal care sessions, or user pain-report data corresponding to said one or more personal care sessions.

The method 10 further comprises determining 18 a second metric of user pain experienced during the one or more personal care sessions based on the further data;

The method 10 further comprises deriving 20 a pain calibration metric based on a relationship between the first and second metrics of estimated user pain.

Preferably the method further comprises generating a data item indicative of the pain calibration metric.

The method may further comprise generating a report based on the data item and transmitting the report via a communication channel to a remote computer, for example a computer accessible to a practitioner such as a dental practitioner. For example, the report may be uploaded to a server, for example a cloud-based server or other server which is accessible to the practitioner. This way, the practitioner can be informed as to the pain status of the patient

As noted above, the method can also be embodied in hardware form, for example in the form of a processing device which is configured to carry out a method in accordance with any example or embodiment described in this document, or in accordance with any claim of this application.

To further aid understanding, Fig. 4 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system does not have to comprise all of the illustrated hardware components; it may just comprise a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface. In the illustrated example of Fig. 4, the system 30 further comprises one or more sensors 42 for measuring sensing data indicative of personal care action by the user using the device over the course of one or more personal care sessions. The one or more sensors 42 are integrated in a personal care device 52 for use by the user. The personal care device may be part of the system 30 in some embodiments.

The one or more sensors 42 may include one or more force and/or motion sensors for sensing motion of the device and/or forces exerted on or by the device. Additionally or alternatively, data may be used from one or more operational signal sensors integrated in the personal care device 52 adapted to output an operational signal indicative of an operational parameter of the personal care device which correlates with physical interaction of the personal care device with bodily surfaces. An example of an operational parameter is an electrical drive signal associated with an actuation device which actuates a portion of the personal care device, e.g. a drive train mechanism which drives oscillation of a powered toothbrush or shaver, or a motor mechanism which drives a massage device. The electrical characteristics of the drive signal can change as a function of applied pressure since applied pressure exerts more physical resistance on the actuation mechanism. Another example of an operational parameter is jet flow of an oral irrigation device or powered flossing device.

In this example, the system 30 further comprises one or more biological parameter sensors 46 for generating sensor data indicative of a biological parameter of the user over the course of one or more personal care sessions. Although this is shown in Fig. 4 as separate from the personal care device 52, optionally it may be integrated in the personal care device, e.g. a skin conductance sensor integrated in the handle of the device.

With regards to the physiological or biological sensor measurements, there are a variety of different options. For example, skin conductance sensors, such as galvanic skin response (GSR) sensors, are known to provide an output which correlates with pain-related arousal. Thus a signal level as a function of time of a skin conductance sensor could in some embodiments be used as the pain signal as a function of time.

Another biological parameter measurement which is known to correlate with pain is heart rate variability. Reference is made for example to the paper: Forte, G., Troisi, G., Pazzaglia, M., Pascalis, V. D., & Casagrande, M. (2022). Heart Rate Variability and Pain: A Systematic Review. Brain Sciences, 12(2), 153.

Heart rate variability can be derived from a heart rate sensor signal, such as from a PPG sensor signal.

Another biological parameter measurement which is known to correlate with pain is breathing or respiration rate. Thus a respiration rate sensor could be used in some examples. For example, respiration rate can be derived from a PPG sensor signal.

Another biological parameter measurement which is known to correlate with pain is facial expression. Facial expression data could be obtained from processing of camera image data.

One or more biological sensors could be integrated in the personal care device 52 itself. For example, a skin conductance sensor could be integrated in the handle portion of a personal care device, such as the handle portion of an oral care device, such as the handle portion of a powered toothbrush. Likewise, a PPG sensor could additionally or alternatively be integrated in the handle portion of a personal care device such as a shaver or powered toothbrush. A PPG sensor can be used to measure heart rate, heart rate variability and/or respiration rate. Alternatively, a separate one or more biological sensing units could be provided. Alternatively, one or more biological sensors could be integrated in a wearable unit, such as a wristband or patch. In some embodiments, the processing device could also be integrated in the same wearable unit.

With regards to the user pain report data, this may, by way of one example, comprise visual analog scale (VAS) user report data. In this regard, reference is made to the paper: Langley, G. B., & Sheppeard, H. (1985). The visual analogue scale: its use in pain measurement. Rheumatology international, 5(4), 145-148.

In some embodiments, the processing device 32 is integrated in the personal care device 52. In further embodiments, the processing device 32 may be comprised by a computing device external to the personal care device, the computing device communicatively coupled with the personal care device or with a datastore with which the personal care device is communicatively coupled. In further embodiments, the processing device 32 may be a cloud-based processing device.

One application area is in the domain of oral care. In some embodiments, the personal care device 52 is an oral care device, the aforementioned personal care action is cleaning action, and the personal care session is an oral cleaning session. In further embodiments, the personal care device may be a skin care device, e.g. a light therapy device, and the personal care action is skin care action and the personal care session is a skin care session. In further embodiments, the personal care device is a grooming device, e.g. a shaver, and the personal care action is a grooming action (e.g. shaving), and the personal care session is a grooming session (e.g. shaving). In further embodiments, the personal care device is a muscle/joint care device (e.g. a massage device). In each case, the personal care device is a device for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through monitoring patterns in the sensor data.

As noted, the one or more sensors may include one or more force and/or motion sensors, and/or one or more operational signal sensors adapted to output an operational signal indicative of an operational parameter of the personal care device which correlates with physical interaction of the personal care device with bodily surfaces. An example of an operational parameter is an electrical drive signal associated with an actuation device which actuates a portion of the personal care device designed for making contact with the user in use, e.g. a drive train mechanism which drives oscillation of a body-contacting portion of a powered toothbrush or shaver, or a motor mechanism which drives a body-contacting portion of a massage device. The electrical characteristics of the drive signal can change as a function of applied pressure since applied pressure exerts more physical resistance on the actuation mechanism. Another example of an operational parameter is jet flow of an oral irrigation device or powered flossing device.

It is noted that integrated force and/or motion sensors might be sensors that are also utilized for other sensing purposes, such as monitoring user brushing behavior (in the case of oral care) in order to provide feedback and advice on brushing coverage and brushing technique. Thus, in practice, the sensors that are used according to the proposed inventive concepts might already be integrated in the personal care device. The inventive concept could even be enabled in some embodiments purely through a software update, utilizing existing hardware in the personal care device.

To illustrate and exemplify the inventive concepts, examples are presented below with reference to an oral care device, for which the aforementioned personal care action is cleaning action, and the personal care session is an oral cleaning session. However, it will be recognized that the principles outlined are easily applicable to other types of personal care device, such as, by way of example, a skin care device, e.g. a light therapy device, a grooming device, e.g. a shaver, or a muscle/joint care device (e.g. a massage device). With any personal care device, the device is typically designed for application to an area of the user's body for a care function, whereby physical stimulation of one or more areas of the body is caused by such application during the care session. Thus, the normal use of the device inherently entails a systematic physical probing of one or more areas of the body, whereby a pain response may be elicited and can be measured through monitoring patterns in the sensor data. Thus it can be seen how the principles of the general inventive concept outlined above, and described below, can be used in any personal care device. Hence, reference in the below-described embodiments to an oral care device may be replaced by reference to any other personal care device without substantive change to the inventive principles. Likewise, reference to cleaning action can be replaced by reference to personal care action and reference to a cleaning session can be replaced by reference to a personal care session.

In the case that the personal care device is an oral care device, the oral care device may be a powered toothbrush in some embodiments. The toothbrush may have a brush head carrying an array of cleaning elements such as bristles. Integrated force and/or motion sensors may include a pressure sensor for sensing an application pressure of the cleaning elements of the brush head to teeth during operation. Such a pressure sensor could be integrated in the brush head itself, but more usually would be integrated in the handle portion of the toothbrush, to which the brush head is attached. The brush head may be removably attached to the handle portion to enable replacement. Application pressure to teeth can be sensed e.g. via a cantilever pressure sensing approach.

Integrated force and/or motion sensors may include a motion sensor for sensing motion of the personal care device. The motion sensor may comprise or take the form of an inertial measurement unit (IMU) comprising typically one or more accelerometers, for example a tri-axial accelerometer arrangement.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

The pain experienced by a patient at home can be an important source of information for a clinical practitioner. In particular, it provides an indicator of potential tissue health or pathology. For example, if the information is exported to the clinical practitioner, it might provide a prompt for the practitioner to contact the patient. However, it is difficult to accurately establish a pain response because it is subject to many variable factors including, mainly, the patient's pain sensitivity and reactivity.

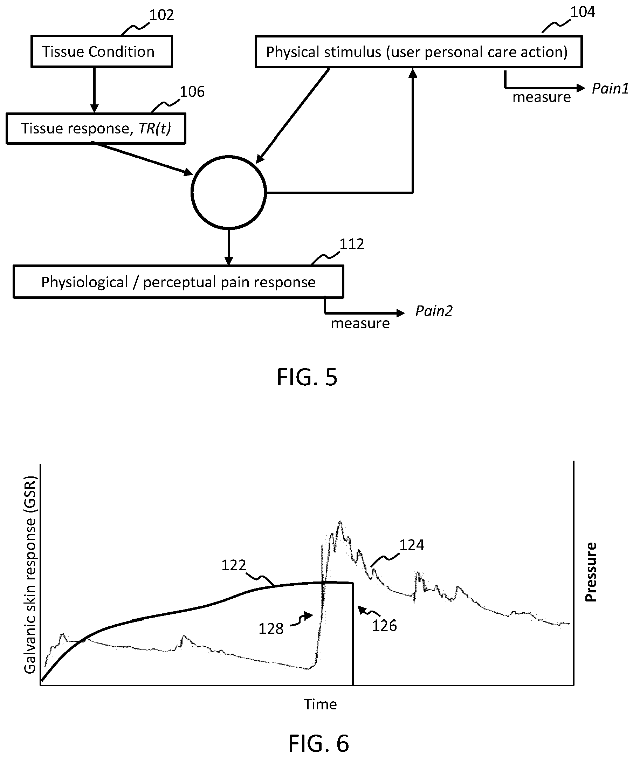

To help illustrate the concepts underlying the present invention, Fig. 5 provides a schematic illustration of pain signal and pain response pathways within the context of application of a personal care device to a body area during a personal care session. By way of example, this might involve application of a toothbrush to teeth, or application of a skincare or grooming device to skin.

Fig. 5 shows a tissue condition $TC(t)$ parameter 102 corresponding to a status of the tissue at an application location of the personal care device at measurement time t. The status of the tissue means the health or pathology status of the tissue e.g. healthy tissue, wounded tissue, etc.

Fig. 5 further illustrates application of a physical stimulus 104 to the tissue site, in the form of personal care action by the user, i.e. application of the personal care device to the tissue site for the purposes of personal care action. In the context of embodiments of the present invention, this personal care action by the user, involving engagement of the personal care device with the tissue site, is measured via the previously discussed sensor data 44 from one or more sensors 42 integrated in a personal care device 52 indicative of personal care action by the user using the device over the course of one or more personal care sessions (see Fig. 4).

Fig. 5 illustrates the measurement of a first pain signal $pain1(t)$ measured as function of time, t. The first pain signal is derived from patterns in said sensor data obtained from said one or more sensors integrated in the personal care device indicative of personal care action by the user using the device. For example, it may correspond to an application pressure of the personal care device to body surfaces or scrubbing intensity of the device exerted by the user. Furthermore, in view of the fact that *pain1(t)* corresponds to a parameter of care action by the user, it also provides a correlate of the physical stimulus being applied to the tissue site.

A neural response 106 to applied physical stimuli, at the level of the tissue, is represented as an objective tissue response parameter *TR(t)*. This neural tissue response is a function of the tissue condition, *TC,* and a function of the applied physical stimulus (of which *pain1(t)* is a proxy indicator). Here it is assumed (for simplicity) no time delay. Thus *TR(t)* can be understood as a function $f_{TR}$ of *TC(t)* and *pain1(t)*.

The objective tissue neural response *TR(t)* leads to a measurable physiological or perceptual pain response 112, which is measured as signal *pain2(t+$\tau_1$)* with a time delay $\tau_1$. The parameter *pain2(t)* corresponds to the second pain metric referred to previously. Hence, the physiological or perceptual pain response *pain2(t)* at time *t+ $\tau_1$* can be understood as an invertible function $f_{PR}$ of the tissue response *TR* at time *t.*

The objective tissue response, TR, also results in the user adapting the applied stimulus, i.e. *pain1(t),* due to an experienced pain or due to a reflex action. This occurs with a certain reaction time $\tau_2$. Hence, the stimulus *pain1(t)* at time *t* is some function $f_{pain1}$ of the tissue response *TR* at time *t- $\tau_2$*. Here, $f_{pain1}$ implicitly incorporates subjective factors, such as the person's pain tolerance or threshold.

Based on the above, an equation for the measured physiological or perceptual pain response *pain2(t)* gives:

$$pain2(t+\tau_1) = f_{PR}(TR(t)) \rightarrow TR(t-\tau_2) = g_{PR}(pain2(t+\tau_1-\tau_2)),$$

where $g_{PR}$ is an inverse function of $f_{PR}$. Inserting this result into the equation for *pain1(t)* then yields:

$$pain1(t) = f_{pain1}(TR(t-\tau_2)) \rightarrow pain1(t) = f_{pain1}(g_{PR}(pain2(t+\tau_1-\tau_2))).$$

This may be rewritten as:

$$pain1(t) = T(pain2(t-\tau)),$$

with $\tau = \tau_2 - \tau_1$, and where *T* is a transfer function between the first pain metric *pain1(t)* and a second pain metric in the form of the measured physiological or perceptual pain response, *pain2(t).*

In the above model, both $f_{PR}$ and $f_{pain1}$ are functions of the tissue pain response TR, but it is assumed that $f_{pain1}$ is more indicative of pain sensitivity, namely the proneness of a person to react to a pain stimulus, since this provides the mapping to the physical behavior of the user in terms of action with the personal care device.

As discussed above, in accordance with embodiments of the present invention, a calibration metric is derived, based on a relationship between first and second metrics of user pain. In accordance with at least one set of embodiments, deriving the calibration metric may mean deriving the transfer function T, or an approximation thereof. In practice it may not always be possible to fully reconstruct the transfer function and so approximations for calibration are proposed.

By way of further general illustration of the principles motivating embodiments of the invention, Fig. 6 shows a simulated graph indicating a first 122 pain signal, representative of an application pressure by a user of a personal care device on a body area, and a second pain signal 124 indicative of a GSR signal for the same user over the same time period.

Here, a sudden rise 128 in the GSR signal 124 indicates that the user has experienced an acute pain sensation. Typically, the GSR response is measured around one second after the acute pain is experienced. At a temporally correlated moment, there is a rapid reduction 128 in application pressure 122 of the personal care device, in this case rapidly breaking contact of the device with the tissue. The application pressure drop 126 occurs with a short time delay following the rise in the GSR signal. Although both correspond to the same pain-inducing stimulus. This graph is intended to illustrate the concept of how two different measurements can provide signals of pain events, and which are correlated to one another with a particular calibration. Here, there is a short time delay between the two pain measures. There is also naturally a certain calibration between the magnitude of response in each signal. It is the subject of the present application to find a metric which provides an indication of at least one aspect of the calibration between two pain measures.

With reference to Fig. 6, it is noted that in some cases, e.g. with a relatively light-weight personal care device, such as a toothbrush, the response time of the user to the acute pain may well be faster than the GSR response time, whereby the device response may precede the physiological response. It is also notable that the GSR signal 124 drops after the application pressure 122 is relieved. Therefore, it can be inferred that the application pressure (proxy for pain) was the cause of the GSR increase.

There will now be outlined a number of example implementations of the inventive concept according to one or more advantageous embodiments.

According to one or more embodiments, the determining the pain calibration metric may comprise determining a transfer function which defines a mapping between the first and second metrics of user pain.

According to one simple set of embodiments, the pain calibration metric may be derived by simply determining a ratio between the first and second metrics of user pain.

This may comprise first deriving the first metric of user pain from a first user pain signal, and deriving the second metric of user pain from a second user pain signal. The first pain signal may be derived from a sensor signal as a function of time over at least one personal care session, and the second pain signal may be a biological parameter sensor signal as a function of time over at least one personal care session. By way of example, the first pain signal pain1(t) could be taken as pain1(t) = c/P(t), where P(t) is a measure of application pressure of the personal care device to the body as a function of time, and c is a constant (which could in a simple case simple be set equal to 1). This is based on the recognition that pain is inversely correlated with application pressure P(t) because a user will tend to press less hard when they feel pain and vice versa. The second pain signal pain2(t) could be taken as a time-series of measures of the biological parameter, e.g. galvanic skin response (GSR) or heart rate variability (HRV).

By way of example, the first metric of user pain could be computed as a maximum value of the first user pain signal pain1(t), and the second metric of user pain could be computed as a maximum value of the second user pain signal, pain 2(t). In other words, the determining the first metric of user pain may comprise processing the sensor data from the one or more sensors integrated in the personal care device indicative of personal care action by the user to determine a signal of user pain as a function of time over each personal care session. It may further comprise determining a maximum value of said signal of user pain for each personal care session, and using said maximum value or an average thereof over the one or more personal care sessions as the first metric of user pain. The determining the second metric of user pain may comprise processing the biological parameter sensor data to determine a further signal of user pain as a function of time over each personal care session, and determining a maximum value of said further signal of user pain for each personal care session, and using said maximum value or an average thereof over the one or more care sessions as the second metric of user pain.

To explain, the time shift t between the first and second user pain signals is a priori not known. It depends on the subject's reaction time in adapting the personal care action in response to the pain, as represented in the first pain signal, as well as the delay time of the physiological or perceptual pain response, as represented in the second pain signal. Thus, it is a challenge to directly relate one pain measure which is a function of time to another which is a function of time. Furthermore, in the case that the second pain metric corresponds to user pain-report data for said one or more personal care sessions, there may not in fact be any time-dependent measure for the pain response. Thus a simple way to compute a relationship between the first and second pain measures is to compute a maximum value of the first pain signal over a single session (or an average thereof over multiple personal care sessions), and a maximum value of the second pain signal over a single session (or an average thereof over multiple personal care sessions), and to derive a relationship between the two maximum values, e.g. a ratio between the two.

According to a further example set of embodiments, instead of using a maximum value of the first and second pain signals, average values might be used over one personal care session or over multiple personal care sessions.

For example, stated more precisely, in some embodiments, the determining the first metric of user pain may comprise processing the sensor data to determine a signal of user pain as a function of time over each personal care session, and determining an average value of said signal of user pain for each personal care session, and using said average value as the first metric of user pain. The determining the second metric of user pain may comprise processing the biological parameter sensor data to determine a further signal of user pain as a function of time over each personal care session, and determining an average value of said further signal of user pain for each personal care session, and using said average value over the one or more care sessions as the second metric of user pain.

Thus, as a result, here it is proposed to compare the average first pain signal values to the average second pain signal values over one session. A ratio between the two can be computed as the calibration metric. This ratio can be used in future sessions with the personal care device in order to interpret the subjective or proxy signals with respect to the pain experience of the user. For, example, in future personal care sessions, in the event that only one signal is present, the calibration metric can be used to better interpret this signal in terms of actual pain response, or calculate or reconstruct the other pain metric, thereby deriving a more all-round picture of the pain response.

In cases where the tissue condition of the user can be assumed to be mainly good across the mouth, with only wounds/damaged tissue at limited locations, this computation of the calibration metric provides a good representation of the status of the relevant tissue. The computation of the average may comprise computing a mean value or a different average measure, such as for instance a median value. The median gives less weight to outliers in the resulting pain metrics, hence potentially yielding a more representative value for the calibration metric. Additionally, by taking the average or median over a session, the resulting pain metrics are insensitive to the (possibly varying) time delay t between the time-dependent pain signals upon which the pain metrics are based.

In relation to either of the above two examples for the calibration metric, instead of computing the calibration metric based on data from a single personal care session, longitudinal assessment using data acquired over multiple sessions may be considered. This can provide a more accurate calibration metric which evens out any local temporal variations in the measured parameters.

As explained above, the first pain metric, *pain1*, and second pain metric, *pain2,* are related via a transfer function *T,* where there is additionally a time delay $\tau$ between the two: *pain1(t) = T(pain2(t- $\tau$)).* This time delay is a result of the reaction time of the user in adapting the personal care action (as reflected in *pain1*) to the experienced pain, as well as the delay time of the (physiological or perceptual) pain response (as reflected *in pain 2*). The transfer function may be estimated more precisely by first solving the unknown time delay r. This can be done by applying cross-correlation C, with time integration over a single person care session as follows:

$$C(\Delta) = \int_{session} pain1(t)\,pain2(t - \Delta)\,dt$$

The time delay $\tau$ then equals the value $\Delta$ for which the cross-correlation *C($\Delta$)* is found to be maximum.

For example, in some embodiments, the determining the first metric of user pain comprises processing the sensor data to determine a signal of user pain as a function of time over each personal care session and the determining the second metric of user pain comprises processing the sensor data to determine a further signal of user pain as a function of time over each personal care session. The determining the calibration metric comprises, for at least one of the one or more personal care sessions, computing a cross-correlation between the first and second signals of user pain and determining from the cross-correlation a time offset between the first and second pain signals.

Once the time offset has been computed, it may be used to correct for the time offset. For example, the method may further comprise adjusting a time-registration between the first and second pain signals to compensate for the determined time offset.

In some embodiments, position or location information may be taken into account to provide a more precise pain calibration metric. This may allow for a spatially resolved pain calibration metric, or may allow for a single overall pain calibration metric which has been averaged over multiple different positions to obtain a more representative value for the subject.

For example, the method may further comprise receiving position sensor data, and wherein the method comprises determining a respective first and second metric of estimated user pain for different respective bodily locations.

The method may further comprise computing an average of the first pain metrics for the different locations and an average of the second pain metrics for the different locations, and computing the calibration metric based on a relationship between said average first pain metric and average second pain metric.

Additionally or alternatively, the method may further comprise computing a respective calibration metric for said different respective bodily locations.

In some embodiments, computing the first and second pain metrics and/or the pain calibration metric may involve one or more additional pre-processing steps. For example, in some embodiments, each of the first and second pain metrics are extracted from first and second time-dependent pain signals. In some embodiments, each of the first and second pain signals might be normalized before extracting the first and second pain metrics. In some embodiments, each of the first and second pain signals might be first processed to remove the DC bias component of the signal, leaving just the AC component. For example, the signals might each be high-pass filtered.

In some embodiments, the first pain metric may be a time-dependent pain signal, and the second pain metric might be time-dependent pain signal, and wherein a calibration metric is computed based on computing an inner product of the two pain signals.

In some embodiments, the first pain metric may be a time-dependent pain signal, pain1(t), and the second pain metric might be time-dependent pain signal, pain2(t), and wherein the first pain metric pain1(t) is modelled as the convolution of an impulse response, I, with metric2, pain2(t), or vice versa. In this case, the deriving the calibration metric may comprise deriving the impulse response. This allows for deriving a calibration metric between two time-dependent pain metrics which preserves time-dependent calibration information between the two.

To explain this further, mathematically, it is possible to represent the relationship between the first and second pain metrics using the general formula for convolution as follows:

$$pain1(t) = I(t) * pain2(t) \quad \rightarrow \quad pain1(t) = \int_{-\infty}^{\infty} I(\Delta)\,pain2(t - \Delta)\,d\Delta$$

where I is the impulse response (and * is the common notation indicating convolution).

As mentioned above, pain1(t) at time t may be understood as depending on pain2(t) over a time period up to (*t-τ*). Thus, the interval of the integration may be limited:

$$pain1(t) = \int_{\tau}^{\infty} I(\Delta)\, pain2(t - \Delta)\, d\Delta$$

Furthermore, pain1(t) at time t will, in reality, not be related to pain2(t) over an infinite period of time before t, and thus, practically, the upper bound of the integration interval may be set at a finite value instead of infinity (e.g. corresponding to the start of the personal care session).

When pain1(t) and pain2(t) are both measured, the impulse response may be retrieved using deconvolution techniques.

The derivation of the impulse response provides the most high-resolution information about the relationship between pain1(t) and pain2(t), but is computationally demanding. Thus, where it is preferred to limit computational resource, the simplified techniques already discussed above, in which single-value calibration metrics are derived, may be used.

In above-described examples, the calibration metric is obtained through processing performed in the time domain. According to one or more further embodiments, a first and second pain metric might be derived in the frequency domain, and wherein the calibration metric is computed based on a relationship between the first and second pain metrics in the frequency domain. For example, a first time-dependent pain signal might be obtained based on processing of the personal care device sensor data, and second time-dependent pain signal obtained from the further data (e.g. biosensor data), and wherein each of the time-dependent signals is transformed into the frequency domain, and wherein the calibration metric is derived from a relationship between the two signals in the frequency domain.

For example, the first and second pain metrics, pain1(t) and pain2(t), measured in the time domain *t,* can be transformed to the frequency domain by applying either a Laplace or Fourier transform. When in the time domain, pain1(t) could be understood as corresponding to the convolution of an impulse response *I* with pain2(t). In the frequency domain, pain1(t) may be understood simply as the multiplication of a frequency response *F* with pain2(t). Here *F* equals the Laplace or Fourier transform of the impulse response *I*.

This might be expressed formally as follows:

$$pain1(t) = I(t) * pain2(t) \longleftrightarrow pain1(\omega) = F(\omega)\, pain2(\omega)$$

where $\omega$ is the frequency. The frequency response *F* in this case may be understood as the transfer function (calibration function) between the first and second pain metric, and is thus given by division of pain1(t) and pain2(t) when in frequency domain.

In some embodiments, the steps of the method of Fig. 3 might be repeated at regular time intervals to account for any variations in the calibration metric. The calibration metric is thus regularly updated over time.

The derived calibration metric has a variety of useful technical applications. In a most general sense, it will be recognized that a metric which is a function of two different measures of user pain, each being generated through a different neural response pathway/mechanism, provides a biologically informative parameter. As explained above, since pain is an indicator of tissue pathology, a pain-related parameter which is a function of two pain measures, each containing information related to different pain-response mechanisms, can help in giving a more objective and robust indicator of underlying tissue health or pathology.

How the calibration parameter is used would depend upon the intended effect.

For example, as has been explained above, a person's pain sensitivity is more reflected by the first pain metric, derived from the sensor data indicative of user response actions to experienced pain. For a certain fixed magnitude of the second pain metric, a higher correlated response of the first pain metric would be indicative of higher pain sensitivity. Thus, one could say that pain sensitivity is correlated with the ratio of pain1:pain2 (where pain1 is the first pain metric and pain2 is the second pain metric). Thus, this information can be used in a range of different scenarios to modify characteristics of potentially pain-inducing operations to account for a patient's known higher pain sensitivity.

To explain and illustrate with greater clarity, some example applications or uses of the calibration metric will now be described.

According to at least one set of embodiments, it is envisaged that the derived calibration metric might be stored in a datastore for later recall and used in modulating or calibrating a pain measure derived in a subsequent session. This might be a subsequent personal care session using the same or a different personal care device. A single pain measure might be derived, using only one of the two modalities utilized in computing the pain calibration metric, and wherein the pain calibration metric can be applied to this pain measure.

For example, to state this more explicitly, an aspect of the invention is a method for calibrating pain measurement information for a user. This may comprise receiving input data, the input data comprising biological parameter sensor data acquired during use of a personal care device over the course of one or more personal care sessions, or user pain-report data corresponding to said one or more personal care sessions, and determining a measure of estimated user pain experienced during the one or more personal care session based on the input data. Additionally or alternatively, the method may comprise receiving input data, the input data comprising sensor data from one or more sensors integrated in a personal care device indicative of personal care action by the user using the device over the course of one or more personal care sessions, and determining a measure of estimated user pain experienced during the one or more personal care session based on analysis of patterns in the sensor data.

The method further comprises retrieving from a datastore a pre-derived pain calibration metric for the user. The method further comprises applying the pain calibration metric to the measure of estimated user pain to derive a calibrated measure of estimated user pain. The method further comprises generating a data item indicative of the calibrated measure of estimated user pain.

The above method can be provided as a separate aspect of the invention, or its steps may be applied as an embodiment of the previously discussed methods for generating the calibration metric.

By way of further example, the pre-derived pain calibration metric may be used to adapt a mode of operation of a personal health care device. For example, it is desirable to set a brushing intensity so as to minimize pain to a user. A user's personal pain calibration metric provides a means for assisting in this. In a simple example, the brushing intensity might be adjusted in dependence upon the pain calibration metric. For example, it might be adjusted in dependence upon a ratio of the first pain metric to the second pain metric. As explained above, this ratio approximately correlates with pain sensitivity. Thus, reducing brushing intensity for higher pain1:pain 2 ratio would account for greater sensitivity.

By way of example, a method may be provided comprising retrieving from a datastore a pre-derived pain calibration metric for the user and using this as an input to an algorithm which controls operation of a personal care device, and wherein a mode of operation is adjusted dependent on the pain calibration metric. For example, brushing intensity could be adjusted to a lower intensity as a function of the pain calibration metric. As a further example, the personal care device may include one or more sensors, and may be adapted for deriving one of the two pain metrics, and wherein the pain calibration metric is used to modulate or adjust the derived pain metric.

By way of a further example, the pre-derived pain calibration metric may be used to determine a measure of medication effectiveness, wherein the medication has an anesthetic function. This can be done by determining a value for the pain calibration metric before anesthetic administration (i.e. in normal circumstances) and afterward and comparing the two. To explain further: anesthetics have less effect on physiological responses than they have on behavioral responses. Thus, in practice, after administration of an anesthetic, the expectation would be that a patient's observable behavior would correspond to that in the absence of pain, while the biological signal responses would remain unchanged. This would have a resultant effect on the calibration metric relating the two pain measures. For example, if a patient receives anesthetics during a dental procedure, after the dental procedure the patient can brush (at least a region of) their teeth again. In this circumstance, it is expected that the physical source of pain at the tissue site would still be present, i.e. the neural source of pain is present, but the pathways to perception and behavioral response are inhibited. However, one would at the same time expect the physiological systems normally correlated with pain, e.g. skin conductance, to still reflect presence of pain. Thus, by re-measuring the pain calibration metric after administration of anesthetic, and comparing it against a value for the pain calibration metric acquired before anesthetic, a change in the values can provide an indication of the effectiveness of the anesthetic.

By way of further example, the pre-derived pain calibration metric might be used to configure personalized drug dose delivery. Here a patient may be continuously monitored, and wherein the second pain metric is continuously measured (e.g. using a biological parameter sensor, and/or through regular collection of pain self-report data). The measure of the second pain metric could then be modified or transformed based on the pre-derived pain calibration metric for the patient. The dosage setting of the drug delivery mechanism may be adjusted in dependence upon this transformed pain measure.

There are different approaches to performing the analysis of patterns in the sensor data in order to determine the first pain metric.

The first and second pain metrics can either each be a single-value (scalar) metric or can be a function of time. In the case that it is a single value metric, it can be derived in some examples by first obtaining a pain signal which is a function of time (e.g. GSR or inverse of application pressure) and then extracting or synthesizing from that a single value metric (e.g. an average or maximum value). With regards to the first pain metric in particular, in some examples, obtaining a pain signal which is a function of time may be done simply through measuring a time series of values from a sensor over a time period, or it may be constructed. In the latter case, for example, deriving the first pain metric may comprise: detecting occurrence one or more discrete pain events over a personal care session based on detecting one or more pre-defined waveform features (fingerprints) in a sensor waveform; constructing a pain signal by plotting the pain events as a function of time to form a pain event time series. Each pain event may have an associated time and magnitude. This constructed pain signal could be used as the first pain metric. Alternatively, from this might be extracted a single-value pain metric (e.g. from an average or maximum value of the plotted pain event points).

According to one approach, the obtained sensor data includes a pressure sensor waveform indicative of application pressure of a portion of a personal care device to surfaces during use, and wherein the first pain metric is determined based on the pressure signal. For example, if the personal care device is an oral care device, e.g. a powered toothbrush device, the pressure sensor waveform may be indicative of application pressure of the brush head cleaning elements (e.g. bristles) to tooth/oral surfaces. Such pressure sensor data provides information indicative of cleaning action by the user using the device over the course of one or more oral cleaning sessions because it relates to how hard the user is pressing the cleaning elements onto the oral/dental surfaces during the cleaning session. Likewise, if the personal care device is grooming device, e.g. a shaver, the pressure sensor waveform may be indicative of application pressure of the shaver cutting area to the skin of the user during use. Such pressure sensor data provides information indicative of shaving action by the user over the course of one or more shaving sessions. Similar principles apply for other types of personal care device.

Application pressure sensor data is useful for inferring information indicative of user pain during a personal care session because it is known that the application pressure will be inversely related to the level of pain experienced (e.g. the brushing pressure reduces as the pain increases). Thus, analysis of the pattern of pressure over the course of a single personal care cycle, or multiple cycles, can provide information about user pain experience.

In one simple example, the first pain metric may be derived from a first pain signal, where the first pain signal is inversely proportional to the measured application pressure. In particular, the first pain signal, $pain1(t)$, could be taken as $pain1(t) = c/P(t)$, where $P(t)$ is a measure of application pressure of the personal care device to the body as a function of time, and $c$ is a constant (which could in a simple case simply be set equal to 1). This is based on the recognition that pain is inversely correlated with application pressure $P(t)$ because a user will tend to press less hard when they feel pain and vice versa. The first pain metric may be a function of time, in which case it might be set equal to this pain signal. Alternatively, the first pain metric may be extracted or synthesized from the pain signal, e.g. using any of the methods already described above, e.g. by extracting a maximum value or average value over a personal care session.

By way of a further example, the observation of the application pressure, e.g. brushing pressure, during the entire personal care cycle, e.g. cleaning cycle, will show a pattern whereby differences in spread of application pressures as a function of time or location (for example compared to the baseline measurements) will provide an indication of pain: namely if unexpected drop in pressure is seen in the pattern (where normally a fairly constant pressure would be observed), this may be an indication of a pain-causing abnormality in the lower-pressure area. Even more simply than this, a standard threshold pressure could be set, and wherein any drop in applied pressure below this threshold over a section of the sampled signal is taken as an indication of a pain experience (pain event) in the location of the anatomy (e.g. mouth) to which that signal section corresponds. A record of such pain events during a personal care session may be used to derive the first pain metric.

In relation to this set of embodiments, as a general approach, it could be stated that the first metric during each of the one or more personal care sessions can be determined further based on reference sensor pattern information associated with user pain or user comfort. The reference sensor pattern information could include a reference pattern of baseline brushing pressure as a function of time (or location) over the course of a personal care cycle, personalized to the user, and wherein pain in a personal care session is detected by comparing this reference baseline pattern to a pattern measured in the new personal care session. More simply, the reference sensor pattern information may include one or more characteristic pattern features in the sensor signal waveform which are each indicative of either a user pain reaction at a given moment during a personal care session, or user comfort during a given period of a personal care session. For the pressure sensor signal in particular, the relevant characteristic pattern feature would be a valley, or negative spike, in the pressure sensor waveform, indicative of a drop in applied pressure during a sub-interval of a given personal care session. For example, a valley in the signal in which the pressure falls below a pre-defined threshold baseline pressure could indicate a pain event for the sub-interval of the personal care session for which the pressure remains below that threshold. The threshold might be set according to a reference baseline pressure, for example acquired pre-treatment or otherwise during a period or at an anatomy location associated with low or zero pain.

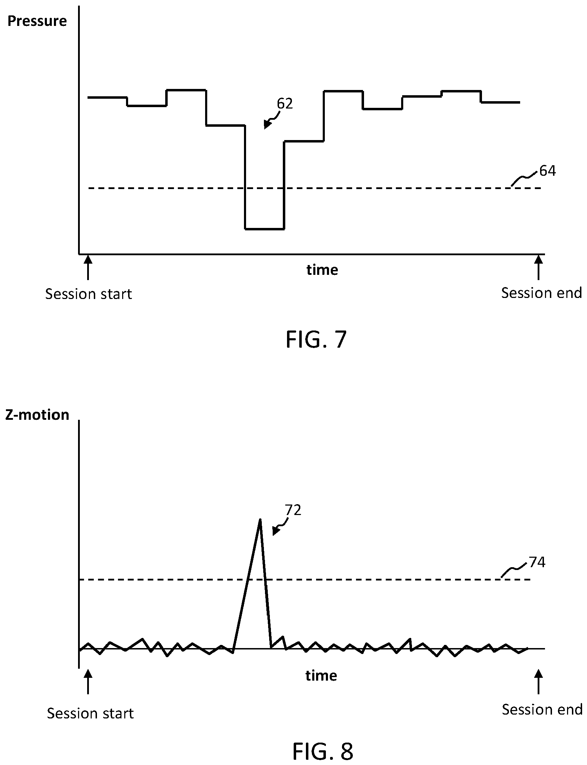

Fig. 7 illustrates an example in the context of an oral care device for performing a cleaning function. Fig. 7 illustrates an example brushing pressure signal as a function of time. A valley in the signal is indicated by arrow 62. During the sub-interval of time spanned by this valley, the applied pressure is below a defined threshold 64 and so it is determined that a pain event occurred during the sub-interval of time spanned by the valley. A valley 62 of depth lower than the threshold 64 represents a characteristic pattern feature whose detection is indicative of pain being experienced for the period of time spanned by the valley. Furthermore, each pain event might have an associated magnitude, e.g. defined in dependence upon an amplitude of the relevant waveform feature, e.g. an amplitude of the valley 62 in this example.

Optionally, in some embodiments, the pressure signal as a function of time or location in the mouth could be converted into a signal indicative of user pain as a function of time or location over the course of each of the one or more cleaning sessions. In a simple example, the pressure signal could be used directly as a proxy for pain, since it is expected to be inversely correlated with experienced pain. For example, a first pain signal pain 1(t), could be defined as pain1(t) = c/P(t), where P(t) is a measure of application pressure of the personal care device to the body as a function of time, and c is a constant (which could in a simple case simply be set equal to 1). This can be used as a time-dependent first pain metric, or a single-value first pain metric can be derived from this.

In another example, where discrete pain events are detected by detection of waveform features, in some embodiments a pain signal, pain1(t), could be constructed as a plot of any such detected pain events as a function of time. Each pain event may have a magnitude, e.g. defined in dependence upon an amplitude of the relevant waveform feature, e.g. an amplitude of a valley in the signal 62. In some embodiments, this time-varying plot could be used as a time-dependent first pain metric, or a single-value first pain metric could be extracted from this plot, e.g. as a maximum or average value.

As mentioned above, the brushing pressure signal would be rendered much more specific by addition of location information. The combination of a location sensor and a pressure sensor enables each pain event to be associated with an oral location. This allows a dentist or the user to know whether a pressure drop coincides with the position of a particular dental issue such as a post-treatment wound, whereby the signal can be attributed to pain level with greater certainty.

Thus, in some embodiments, the obtained sensor data may include position sensor data, and wherein the method comprises determining information indicative of user pain as a function of location in the mouth. The determining the measure indicative of user pain as a function of location in the mouth may be based on using the position sensor data to correlate each of the detected valleys in the pressure signal to a corresponding position in the mouth.

Furthermore, it is noted that the utility of a position sensor to correlate pain events with location in the mouth is not confined to embodiments which use a pressure sensor. Regardless of which particular force and/or motion sensor data is used to infer the pain information, the additional use of a location sensor allows characteristic pattern features associated with pain which are detected in the force/motion sensor signal to be correlated with a location in the mouth. Generation of a pain map, as suggested above, is also compatible more widely with any of the other described embodiments in this document.

In accordance with a further approach (which could be either combined with or used in place of the pressure-sensor approach already described), one or more motion sensing means can be used to sense motion of the personal care device. In this case, the sensor signal may show a sudden (impulse) movement of the personal care device as an acute pain is felt. The movement might be e.g. perpendicular to (e.g., away from) the anatomy surface (e.g. tooth surface), but sudden movement in any direction is possible following a feeling of pain. Here, the level of pain can be inferred as increasing as the motion impulse increases.

Thus, according to this approach, the sensor data may include a motion sensor signal, and wherein the first pain metric is determined based on detecting inflections in motion, indicative of user reaction to locally experienced pain.

Thus this approach is also based on detecting one or more characteristic pattern features in the sensor signal waveform indicative of either a user pain reaction at a given moment during a personal care session, or user comfort during a given period of a personal care session. In this approach, the characteristic waveform pattern feature is an inflection or spike or impulse in the signal. Each such detected pattern feature may correspond to a pain event, and in some embodiments a pain signal could be constructed comprised of a plot of any such detected pain events as a function of time. Each pain event may have a magnitude, e.g. defined in dependence upon an amplitude of the relevant waveform feature, e.g. an amplitude of an inflection 62. In some embodiments, this time-varying plot could be used as a time-dependent first pain metric, or a single-value first pain metric could be extracted from this plot, e.g. as a maximum or average value.

The motion sensor could include an inertial sensor such as an accelerometer for example.

Fig. 8 illustrates an example motion sensor signal as a function of time over the course of a single personal care session. The motion sensor in this case is sensitive at least to motion in a direction perpendicular (e.g. away from) the relevant anatomy surfaces (e.g. tooth surfaces) during use of the personal care device (denoted as the z-direction in Fig. 8). An upward inflection or spike or fluctuation in the signal is indicated at arrow 72. The amplitude or height of the inflection or spike or fluctuation in the signal exceeds a threshold motion level 74, which might in this instance correspond to a speed of motion or a rate of acceleration. If an inflection in the signal exceeds the pre-defined threshold 74, it may be classified as corresponding to a pain event.

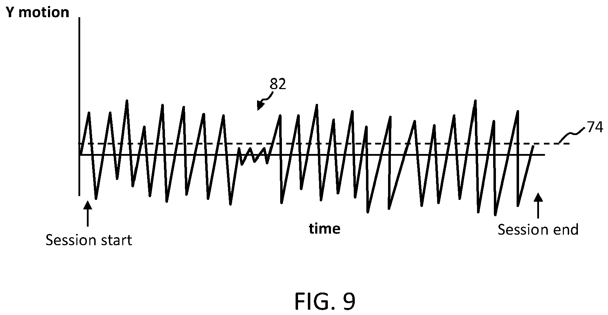

According to a further approach (which may be either combined with or used instead of any of the other approaches in this document), a motion sensor may be used which is operable to detect motion during use of the personal care device. The patterns in this signal are representative of scrubbing action by a user, which manifests as an alternating pattern in the signal waveform. Fig. 9 shows an example. In this example, motion in a direction in-plane with a tooth surface (y-direction) is shown by way of illustration in the graph of Fig. 9. However, scrubbing motion could be sensed with a motion sensor sensing motion in any direction, e.g. simply a tri-axial accelerometer operable to sense motion in any direction.

At locations where pain is experienced, it is to be expected that amplitude of scrubbing motion would decrease compared to that in areas where no pain is felt. Thus here, the sensor data includes a motion sensor signal and a first pain metric may be determined based on detecting in the motion sensor signal periods of alternating motion, indicative of a scrubbing action by the user, and wherein an amplitude or time duration of the periods of alternating motion are used to infer a level of patient comfort.

In particular, each of one or more pain events may be detected by detecting a characteristic waveform feature in the form of a section of an alternating signal having an amplitude which falls below a threshold 74. One example of such a pain event is shown by arrow 82 in Fig. 9. The threshold 74 might be set in some examples as a defined proportion of an average amplitude of the signal over the whole of the personal care session, for example half of the average amplitude. A sub-section 82 of the signal during which the amplitude of a detected alternating motion signal is below the defined threshold may be classified as corresponding to pain events. Again, use of a location sensor in addition may allow these sub-sections of the signal to be correlated with particular locations of the anatomy, e.g. in the mouth, e.g. particular dental sites. Each pain event may have a magnitude, e.g. defined in dependence upon an amplitude of the relevant waveform feature, e.g. an inverse of the amplitude of the lower-pressure scrubbing period 82 in the present example. As in the previous examples, detected pain events could be plotted as a time series to form a time-dependent first pain signal. From this a single value pain metric could be extracted as described previously, or the pain signal could be used as the pain metric itself.

For example, the acquired sensor data can include position sensor data, and wherein the method comprises determining information indicative of user pain as a function of location on/in the anatomy, e.g. in the mouth. User pain as a function of location may be determined based on using the position sensor data to correlate each of the detected signal periods of lower-amplitude alternating motion to a corresponding position on/in the anatomy, e.g. in the mouth.

In addition to or instead of amplitude of the scrubbing motion, also frequency of scrubbing can be used to detect areas of higher pain. In particular, slow careful movements or scrubbing (at that location) are to be expected in areas of higher pain. Thus, in some embodiments, determining a first pain metric may comprise determining a frequency of an alternating motion signal along a direction parallel with application surfaces (e.g. tooth surfaces), identifying continuous motion signal sections for which frequency is below a threshold frequency, and classifying those sections as corresponding to pain events. Again, here the addition of a location sensor will improve specificity. The pain events can be plotted over time to form a time-dependent pain signal. Optionally from this a single-value first pain metric can be derived from the plot in a manner already described above.

In addition to or instead of amplitude and frequency of the scrubbing motion, also the duration of time spent applying the device to a particular area, e.g. duration of time spent cleaning a particular tooth or dental area, could be used to infer a level of pain experienced at that area. Here, the analysis of the sensor signal might comprise first identifying in a force and/or motion sensor signal a plurality of different signal sections corresponding to periods of time spent cleaning different areas, such as different dental areas, such as different teeth. In some cases, this could be done though correlating different signal sections with the output of a motion sensor. In other cases, it could be done through identifying pattern sections separated by breaks or pauses in the signal as the user moves to the next signal. For example for a pressure signal, the pauses might have a recognizable signature or fingerprint, such as being signal periods of a duration being below a threshold duration, and wherein for instance pressure or motion amplitude is below a certain threshold. Once the different signal sections have been identified, a time duration of each one can be determined, and from this it can be determined how long the user spent treating each area, e.g. cleaning each tooth. A short time may indicate a higher pain. A short time combined also with presence during that signal section of one of the characteristic signal waveform features discussed, such as a lower amplitude alternating motion, or a spike in out-of-plane motion, or a dip in pressure, might be used to indicate pain.

Although the above example was described with reference to an example signal corresponding to in-plane motion, in practice the detected alternating motion can be in any direction.

Above have been described example techniques for determining a first pain metric based on identifying certain characteristic pattern features in the sensor signal(s). A further approach, which could be used in combination with or instead of the already described approaches, is to compare all or part of an acquired force and/or motion sensor signal waveform with a personalized reference waveform, acquired at a time or over an area which is known to be associated with low pain. In other words, reference sensor data pattern information is acquired in advance for the user at a time or in a location of the anatomy (e.g. location in the mouth) known to be associated with a baseline pain state, and preferably wherein the baseline pain state is a low pain state. For example, it might be acquired prior to a treatment (e.g. a dental treatment), or prior to emergence of a new complaint. Then, the first pain metric during each of the one or more personal care sessions is determined by comparing a sensor signal for a new personal care session with the reference sensor pattern information associated with user pain or user comfort.

An illustrative example might be implemented as follows, in this case with reference to oral care with an oral care device. First, prior to a dental treatment, or during a calibration period unconnected with any treatment, normal brushing behavior of the user is monitored over a period of time, for instance 3-7 days using the force and/or motion sensors integrated in the oral care device, e.g. powered toothbrush. From this, an average signal waveform as a function of time over a cleaning session may be determined from the acquired data for each of the one or more force and/or motion sensors. From this, a single characteristic or representative value might be obtained. For instance, an average applied pressure. Alternatively, the whole average waveform may be obtained.

After the calibration period, for example after a dental treatment, the force and/or motion sensor data can be monitored in the same way as before, and the recorded signal waveform for each cleaning session can be compared against the reference acquired in the calibration period. Either the whole waveform can be compared against the reference, to therefore detect signal sections which deviate significantly (which could then optionally be correlated to particular mouth locations), or the single characteristic or representative value is compared. Significant deviations may be indicative of a change in pain state.

In other words, any immediate change from the pretreatment pattern is indicative of pain. The first pain metric may therefore be derived based on a deviation of sensor data from the pre-defined pre-treatment baseline. For example, an average application pressure may be determined for each personal care session, and wherein this is compared against a pre-treatment baseline for the application pressure, and wherein the pain metric is set in dependence upon a magnitude of (negative) deviation from this baseline.

Exactly the same principle could be applied for other personal care devices, e.g. skin care device in the context of a skin care treatment, a massage device in the context of a muscle care treatment, etc.

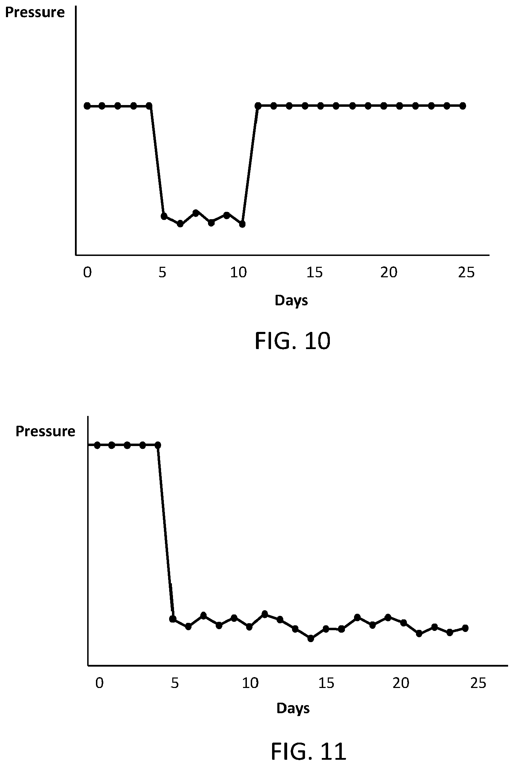

To illustrate visually, Fig. 10 schematically illustrates an example period of 25 days over which oral cleaning sensing data is acquired. The period from day 1 to day 4 is the calibration period. For simplicity, in this example, a single representative value of average brushing pressure was extracted from the brushing pressure waveforms for the cleaning sessions each day. These are plotted as data points on the graph. At day 5, the patient undergoes a dental treatment that leaves a wound at a dental site. It can be seen that average brushing pressure immediately declines. This can be taken as indicative of user pain. The first pain metric for a given personal care session may be set in dependence upon the difference between the average application pressure and the pre-treatment baseline average pressure. The brushing pressure in the example of Fig. 11 varies day-by-day over an approximately 6-day period, while remaining at a level significantly below the reference or baseline level measured during the calibration period in days 1-4. Then, around day 10 or 11, the average pressure recovers to approximately the level prior to the treatment. This indicates that the pain has resolved and the user has returned to normal. It can be inferred that the wound left by the treatment has healed.

Fig. 11 illustrates a comparative example in which the average pressure instead remains around the significantly reduced level following the treatment, and does not recover. This indicates that pain has not resolved, even after 20 days following the treatment. This might indicate that the wound is failing to heal normally.

It is noted that in a more basic implementation, the calibration phase in which the reference waveform or reference value is acquired may not be needed. Instead the reference might be acquired from measuring sensor data at an area known to be associated with a baseline low pain state, such as a different area of the anatomy (e.g. opposite side of the mouth) to the area that was treated or to an area affected by a pathology.

It is further noted that although the examples discussed above are presented in terms of monitoring before and after a treatment, the described approach is applicable in any scenario, whether there is a treatment or not. Pain can be associated with a pathology which is unconnected with any treatment, and a change in the measured sensor data patterns compared to a reference time or a reference area can indicate pain, and thus indicate a change in health status.

As has been noted above, in accordance with any embodiment of the invention, position sensor data indicative of a location or position of an operative part of the personal care device can be acquired and used to register force and/or motion sensor waveforms to position relative to the body/anatomy, e.g. position in the mouth in the case of an oral care device.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing arrangement includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method, comprising:

    obtaining sensor data from one or more sensors integrated in a personal care device indicative of personal care action by the user using the device over the course of one or more personal care sessions;
    determining a first metric of user pain experienced during the one or more personal care sessions based on analysis of patterns in the sensor data;
    receiving further data, the further data comprising biological parameter sensor data acquired over the course of said one or more personal care sessions, or user pain-report data corresponding to said one or more personal care sessions;
    determining a second metric of user pain experienced during the one or more personal care sessions based on the further data;
    deriving a pain calibration metric based on a relationship between the first and second metrics of estimated user pain; and
    generating a data item indicative of the pain calibration metric.

2. The method of claim 1, wherein the determining the pain calibration metric comprises determining a transfer function which defines a mapping between the first and second metrics of user pain.

3. The method of claim 1 or 2, wherein the determining the pain calibration metric comprises determining a ratio between the first and second metrics of user pain.

4. The method of any of claims 1-3,

    wherein determining the first metric of user pain comprises processing the sensor data to determine a signal of user pain as a function of time over each personal care session, and determining a maximum value of said signal of user pain for each personal care session, and using said maximum value or an average thereof over the one or more personal care sessions as the first metric of user pain;
    wherein determining the second metric of estimated user pain comprises processing the biological parameter sensor data to determine a further signal of user pain as a function of time over each personal care session, and determining a maximum value of said further signal of user pain for each personal care session, and using said maximum value or an average thereof over the one or more care sessions as the second metric of user pain.

5. The method of any of claims 1-3, wherein

    wherein determining the first metric of user pain comprises processing the sensor data to determine a signal of user pain as a function of time over each personal care session, and determining an average value of said signal of user pain for each personal care session, and using said average value as the first metric of user pain; wherein determining the second metric of estimated user pain comprises processing the biological parameter sensor data to determine a further signal of user pain as a function of time over each personal care session, and determining an average value of said further signal of user pain for each personal care session, and using said average value over the one or more care sessions as the second metric of user pain.

6. The method of any of claims 1-5,

wherein determining the first metric of user pain comprises processing the sensor data to determine a signal of user pain as a function of time over each personal care session;
wherein determining the second metric of user pain comprises processing the sensor data to determine a further signal of user pain as a function of time over each personal care session;
wherein determining the calibration metric comprises, for at least one of the one or more personal care sessions, computing a cross-correlation between the first and second signals of user pain and determining from the cross-correlation a time offset between the first and second pain signals.

7. The method of claim 6, further comprising adjusting a time-registration between the first and second pain signals to compensate for the determined time offset.

8. The method of any of claims 1-7, wherein the method further comprises receiving position sensor data, and wherein the method comprises determining a respective first and second metric of estimated user pain for different respective bodily locations.

9. The method of claim 8, further comprising computing an average of the first pain metrics for the different locations and an average of the second pain metrics for the different locations, and computing the calibration metric based on a relationship between said average first pain metric and average second pain metric.

10. The method of claim 8, wherein further comprising computing a respective calibration metric for said different respective bodily locations.

11. The method of any of claims 1-10, wherein the biological parameter sensor is a skin conductance sensor, such as a galvanic skin response (GSR) sensor.

12. The method of any of claims 1-11, wherein the one or more sensors integrated in the personal care device include:

one or more force and/or motion sensors; and/or
one or more operational signal sensors adapted to output an operational signal indicative of an operational parameter of the personal care device which correlates with physical interaction of the personal care device with bodily surfaces.

13. The method of any of claims 1-12, wherein the personal care device is an oral care device.

14. A method for calibrating pain measurement information for a user, comprising:

receiving input data, the input data comprising biological parameter sensor data acquired during use of a personal care device over the course of one or more personal care sessions, or user pain-report data corresponding to said one or more personal care sessions, and determining a measure of estimated user pain experienced during the one or more personal care sessions based on the input data, OR
receiving input data, the input data comprising sensor data from one or more sensors integrated in a personal care device indicative of personal care action by the user using the device over the course of one or more personal care sessions, and determining a measure of estimated user pain experienced during the one or more personal care session based on analysis of patterns in the sensor data;

AND
retrieving from a datastore a pre-derived pain calibration metric for the user;
applying the pain calibration metric to the measure of estimated user pain to derive a calibrated measure of estimated user pain;
generating a data item indicative of the calibrated measure of estimated user pain.

**15.** A processing device comprising:

an input/output; and
one or more processors configured to:

obtain sensor data from one or more sensors integrated in a personal care device indicative of personal care action by the user using the device over the course of one or more personal care sessions;
determine a first metric of user pain experienced during the one or more personal care sessions based on analysis of patterns in the sensor data;
receive further data, the further data comprising biological parameter sensor data acquired over the course of said one or more personal care sessions, or user pain-report data corresponding to said one or more personal care sessions;
determine a second metric of user pain experienced during the one or more personal care sessions based on the further data;
derive a pain calibration metric based on a relationship between the first and second metrics of estimated user pain; and
generate a data item indicative of the pain calibration metric and optionally route the data item to the input/output.

**16.** A system, comprising:

a personal care device comprising: one or more sensors configured to generate sensor data indicative of personal care action by the user using the device over the course of one or more personal care sessions; and
a processing device in accordance with claim 15, arranged to receive the generated sensor data as an input at the input/output.

**Pain sensitivity**

Pressure

2

4

## FIG. 1

**Physiological reactivity**

Galvanic skin response

6

8

Time

## FIG. 2

10

| Obtain sensor data from personal care device | 12 |

↓

| Determine first pain metric | 14 |

↓

| Receive bio parameter data | 16 |

↓

| Determine second pain metric | 18 |

↓

| Determine pain calibration metric | 20 |

FIG. 3

32

34

| I/O |

36

| proc |

44

52

Pers. Care device

| sensor(s) | 42 |

48

46

| Bio sensor(s) |

30

FIG. 4

102

Tissue Condition

106

Tissue response, *TR(t)*

104

Physical stimulus (user personal care action)

measure → *Pain1*

112

Physiological / perceptual pain response

measure → *Pain2*

## FIG. 5

Galvanic skin response (GSR)

122

124

128

126

Time

Pressure

## FIG. 6

FIG. 7

FIG. 8

FIG. 9

FIG. 10

FIG. 11

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 22 20 4008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/345950 A1 (ANNONI ELIZABETH MARY [US] ET AL) 11 November 2021 (2021-11-11) * paragraphs [0002], [0025], [0056], [0060], [0076], [0082], [0083], [0087] – [0089]; figures 4A, 7 * | 1-12,15, 16 | INV. A61B5/024 A61B5/11 G16H20/30 A61C17/22 B26B19/38 A61B5/0533 |
| E | WO 2023/006609 A1 (UNILEVER IP HOLDINGS B V [NL]; UNILEVER GLOBAL IP LTD [GB] ET AL.) 2 February 2023 (2023-02-02) * page 8, line 31 – page 9, line 31; claims 1-15; figure 1 * | 1,2,12, 13,15,16 | ADD. A61B5/08 A61N5/06 A61B5/16 A61B5/06 |
| A | US 2018/085584 A1 (THAKUR PRAMODSINGH HIRASINGH [US] ET AL) 29 March 2018 (2018-03-29) * paragraphs [0051], [0068] * | 1-13,15, 16 | |
| A | EP 3 974 126 A1 (KONINKLIJKE PHILIPS NV [NL]) 30 March 2022 (2022-03-30) * paragraph [0025] * | 1-13,15, 16 | |

| TECHNICAL FIELDS SEARCHED (IPC) |
|---|
| A61B A61C B26B G16H |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 15 June 2023 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 22 20 4008

```
Claim(s) completely searchable:
        1-13, 15, 16

Claim(s) not searched:
        14

Reason for the limitation of the search:

The search division has identified multiple independent claims in the
same category, namely method claims 1 and 14, and therefore invited the
applicant to indicate the claims complying with Rule 43(2) EPC on the
basis of which the search is to be carried out (Rule 62a(1) EPC).
In response the applicant indicated independent claim 1. The search has
therefore been limited to independent method claim 1 and independent
apparatus claim 15, as well as claims 2-13 and 16, which depend on claims
1 and 15, respectively.
```

## ANNEX TO THE EUROPEAN SEARCH REPORT
## ON EUROPEAN PATENT APPLICATION NO.

EP 22 20 4008

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2023

| Patent document cited in search report | | Publication date | Patent family member(s) | | Publication date |
|---|---|---|---|---|---|
| US 2021345950 | A1 | 11-11-2021 | US 2018192943 | A1 | 12-07-2018 |
| | | | US 2021345950 | A1 | 11-11-2021 |
| WO 2023006609 | A1 | 02-02-2023 | NONE | | |
| US 2018085584 | A1 | 29-03-2018 | AU 2017334841 | A1 | 16-05-2019 |
| | | | EP 3519037 | A1 | 07-08-2019 |
| | | | US 2018085584 | A1 | 29-03-2018 |
| | | | US 2020188673 | A1 | 18-06-2020 |
| | | | US 2022323760 | A1 | 13-10-2022 |
| | | | WO 2018063912 | A1 | 05-04-2018 |
| EP 3974126 | A1 | 30-03-2022 | CN 116234668 | A | 06-06-2023 |
| | | | EP 3974126 | A1 | 30-03-2022 |
| | | | WO 2022063628 | A1 | 31-03-2022 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CACIOPPO, J. T. ; TASSINARY, L. G. ; BERNT-SON, G. G.** The handbook of psychophysiology. Cambridge University Press, 2007, vol. 44
- **DE RIDDER, D. ; ADHIA, D. ; VANNESTE, S.** The anatomy of pain and suffering in the brain and its clinical implications. *Neuroscience & Biobehavioral Reviews,* 2021, vol. 130, 125-146
- **GARLAND, E. L.** Pain processing in the human nervous system: a selective review of nociceptive and biobehavioral pathways. *Primary Care: Clinics in Office Practice,* 2012, vol. 39 (3), 561-571
- **FORTE, G. ; TROISI, G. ; PAZZAGLIA, M. ; PAS-CALIS, V. D. ; CASAGRANDE, M.** Heart Rate Variability and Pain: A Systematic Review. *Brain Sciences,* 2022, vol. 12 (2), 153
- **LANGLEY, G. B. ; SHEPPEARD, H.** The visual analogue scale: its use in pain measurement. *Rheumatology international,* 1985, vol. 5 (4), 145-148